# EUROPÄISCHE PATENTSCHRIFT

(11) **EP 2 872 478 B1**
(45) Veröffentlichungstag und Bekanntmachung des Hinweises auf die Patenterteilung: **07.12.2016**
(21) Anmeldenummer: 13730491.1
(22) Anmeldetag: 19.06.2013
(51) Int. Cl.: C07C 67/10, C07C 69/24, C07C 1/24, C07C 45/50, C07C 51/235, C07C 11/02, C07C 47/02, C07C 53/126, C07C 51/145, C07C 51/14

(54) **VINVLESTER DER ISONONANSÄURE AUSGEHEND VON 2-ETHYLHEXANOL, VERFAHREN ZU SEINER HERSTELLUNG SOWIE SEINE VERWENDUNG**
VINYL ESTERS OF ISONONANOIC ACID STARTING FROM 2-ETHYL HEXANOL, METHODS FOR THE PRODUCTION THEREOF AND USE THEREOF
ESTER VINYLIQUE DE L'ACIDE ISONANOÏQUE OBTENU À PARTIR DE 2-ÉTHYLHEXANOL, PROCÉDÉ DE PRÉPARATION ET UTILISATION DUDIT ESTER

(30) Priorität: 13.07.2012 DE 102012014396
(43) Veröffentlichungstag der Anmeldung: 20.05.2015
(73) Patentinhaber: OXEA GmbH, 46147 Oberhausen (DE)
(72) Erfinder: JOHNEN, Leif, 46562 Voerde (DE); FREY, Guido, D., 64560 Riedstadt (DE); EISENACHER, Matthias, 46485 Wesel (DE); KOCKRICK, Kristina, 40505 Düsseldorf (DE); STRUTZ, Heinz, 47445 Moers (DE)
(86) Internationale Anmeldenummer: PCT/EP2013/001803
(87) Internationale Veröffentlichungsnummer: WO 2014/008977

(56) Entgegenhaltungen:
- EP-A2- 1 057 525
- EP-A2- 2 404 671
- WO-A1-03/029180
- DE-A1- 19 908 320
- DE-C1- 10 010 771
- G. HÜBNER: "Vinylierung höherer Carbonsäuren an Katalysatorschmelzen", FETTE, SEIFEN, ANSTRICHMITTEL, Bd. 68, Nr. 4, 1966, Seiten 290-292, XP002702538, ISSN: 0015-038X

## Beschreibung

Die vorliegende Erfindung betrifft die Herstellung von einem Vinylester eines Gemisches von Isononansäuren ausgehend von 2-Ethylhexanol durch Dehydratisierung von 2-Ethylhexanol, Umsetzung des erhaltenen Octengemisches zu einem Gemisch von um ein Kohlenstoffatom verlängerten Isononansäuren und anschließende Umsetzung des so hergestellten Isononansäuregemisches zu dem entsprechenden Vinylester.

Vinylester von höheren Carbonsäuren besitzen als Comonomere eine gewisse wirtschaftliche Bedeutung. Mit ihnen können die Eigenschaften von Polymeren, wie beispielsweise Polyvinylchlorid, Polyvinylacetat, Polystyrol oder Polyacrylsäureestern modifiziert werden. So kann beispielsweise die Hydrolysebeständigkeit von Dispersionsanstrichen erhöht werden. Auch für die Herstellung von Klebstoffen werden Vinylester von höheren Carbonsäuren eingesetzt. Als technisch bedeutend für diese Einsatzgebiete haben sich Vinylester auf Basis von 2-Ethylhexansäure, Isononansäure, Laurinsäure oder der Versaticsäuren 911, 10 und 1519 von Shell erwiesen. Diese höheren Carbonsäuren sind beispielsweise durch Oxidation von Aldehyden zugänglich, die durch die Oxoreaktion hergestellt werden, oder durch die sogenannte Koch-Synthese aus dem Olefin, Kohlenmonoxid und Wasser. Bei Vinylestern auf Basis der 2-Ethylhexansäure, Laurinsäure oder Isononansäure, falls die Isononansäure überwiegend aus 3,5,5-Trimethylhexansäure besteht, liegen einheitliche Verbindungen vor, während bei Vinylestern der Versaticsäuren 911 Gemische von hoch verzweigten Carbonsäuren mit 9 bis 11 Kohlenstoffatomen und bei Vinylestern der Versaticsäuren 1519 Gemische von hochverzweigten Carbonsäuren mit 15 bis 19 Kohlenstoffatomen im Vinylester gebunden sind. Bei Vinylestern der Versaticsäure 10 sind strukturverschiedene hoch verzweigte Decansäuren wie Neodecansäuren derivatisiert.

Vinylester können durch Umsetzung der Isononansäuren mit Acetylen, vorzugsweise in Gegenwart von Zinksalzen bei Temperaturen von 200-230°C hergestellt werden (G. Hübner, Fette, Seifen, Anstrichmittel 68, 290 (1966); Ullmanns Encyklopädie der technischen Chemie, 4. Auflage, 1983, Verlag Chemie, Band 23, Seiten 606-607; EP 1 057 525 A2) oder durch die sogenannten Transvinylierungsreaktion mit einem Vinylester einer anderen Carbonsäure, häufig Vinylacetat oder Vinylpropionat, in Gegenwart von Übergangsmetallkatalysatoren (Ullmanns Encyklopädie der technischen Chemie, 4. Auflage, 1983, Verlag Chemie, Band 23, Seiten 606-607; Adelmann, Journal Organic Chemistry, 1949, 14, Seiten 1057-1077; DE 199 08 320 A1, EP 0 497 340 A2, WO2011/139360 A1, WO2011/139361 A1).

Als Rohstoff für die industrielle Herstellung von Isononansäure dient der C4-Schnitt aus der Dampfspaltung von Naphtha. Seine Verfügbarkeit im Vergleich zu den C2-und C3-Spaltprodukten kann durch die Bedingungen der Dampfspaltung gesteuert werden und richtet sich nach den Marktgegebenheiten. Aus den C4-Spaltprodukten wird zunächst 1,3-Butadien durch Extraktion oder durch Selektivhydrierung in n-Butene entfernt. Das erhaltene C4-Raffinat, auch als Raffinat I bezeichnet, enthält überwiegend die ungesättigten Butene Isobuten, 1-Buten und 2-Buten sowie die hydrierten Produkte n-Butan und Isobutan. Aus dem Raffinat I wird im nächsten Schritt Isobuten entfernt und das erhaltene, isobutenfreie C4-Gemisch bezeichnet man als Raffinat II.

Für die Isobutenabtrennung werden in der industriellen Produktion verschiedene Verfahren angewandt, bei denen man die relativ höchste Reaktivität des Isobutens in dem Raffinat I ausnutzt. Bekannt ist die reversible protonenkatalysierte Wasseranlagerung zum tertiär-Butanol oder die Methanolanlagerung zum Methyl-tertiär-butylether. Aus diesen Additionsprodukten kann durch Rückspaltung wieder Isobuten zurückgewonnen werden (Weissermel, Arpe, Industrielle Organische Chemie, VCH Verlagsgesellschaft, 3. Auflage, 1988, Seiten 74-79).

Ebenfalls kann das butadienfreie C4-Raffinat bei erhöhter Temperatur und unter Druck mit einem sauren suspendierten lonenaustauscher in Kontakt gebracht werden. Isobuten oligomerisiert zu Di-isobuten, Tri-isobuten und in geringem Maße zu höheren Oligomeren. Die Oligomere werden von den nicht reagierten C4-Verbindungen abgetrennt. Aus dem Oligomerisat kann dann Di-isobuten oder Tri-isobuten destillativ rein gewonnen werden. Durch die Dimerisierung von n-Butenen mit Isobuten wird in geringem Maße Codimer gebildet (Weissermel, Arpe, Industrielle Organische Chemie, VCH Verlagsgesellschaft, 3. Auflage, 1988, Seite 77; Hydrocarbon Processing, April 1973, Seite 171-173).

Di-isobuten, entweder hergestellt durch die Oligomerisierung von durch Rückspaltung erhaltenem reinen Isobuten oder gewonnen im Zuge der Aufarbeitung eines butadieryfreien Raffinat I, wird anschließend in ein um ein C-Atom verlängertes C9-Derivat überführt. Technisch betrieben wird die Hydroformylierung oder Oxo-Reaktion, bei der Di-isobuten mit Kohlenmonoxid und Wasserstoff in Gegenwart von Rhodium- oder Kobaltkatalysatoren in den entsprechenden Aldehyd umgewandelt werden. Da Di-isobuten in überwiegendem Maße die Octene 2,4,4-Trimethyl-1-penten und 2,4,4-Trimethyl-2-penten enthält, ergibt die Hydroformylierungsreaktion den C9-Aldehyd 3,5,5-Trimethylhexanal als Hauptbestandteil. Weitere C9-lsomere, die in geringen Mengen zugegen sind, sind 3,4,4- und 3,4,5-Trimethylhexanal sowie 2,5,5-Trimethylhexanal, 4,5,5-Trimethylhexanal und 6,6-Dimethylheptanal. Durch Oxidation dieses Aldehydgemisches erhält man eine technisch verfügbare Isononansäure, die üblicherweise einen Gehalt an 3,5,5-Trimethylhexansäure von etwa 90% aufweist (Ullmanns Encyklopädie der technischen Chemie, 4. Auflage, 1975, Verlag Chemie, Band 9, Seiten 143-145; EP 1 854 778 A1).

Di-isobuten kann ebenfalls nach der sogenannten Hydrocarboxylierung oder Koch-Reaktion mit Kohlenmonoxid und Wasser in Gegenwart von Schwefelsäure in die hochverzweigte Isononansäure 2,2,4,4-Tetramethyl-1-pentansäure überführt werden. Aufgrund der zweifachen Alkylverzweigung an dem der Carboxylgruppe benachbarten Kohlenstoffatom wird diese Isononansäure häufig auch als Neononansäure bezeichnet. (Ullmanns Encyklopädie der technischen Chemie, 4. Auflage, 1975, Verlag Chemie, Band 9, Seiten 143-145).

Auch die nach der Isobutenabtrennung im Raffinat II enthaltenen n-Butene werden technisch zu Butenoligomerisaten umgesetzt, aus denen isomere Octene abgetrennt werden, die über die Hydrocarboxylierung in die entsprechenden Isononansäuren überführt werden (DE 199 08 320 A1; EP 1 029 839 A1). Die Oligomerisierung von n-Butenen wird technisch an sauren Katalysatoren wie Zeolithen oder Phosphorsäure auf Trägem betrieben. Hierbei erhält man Octene, die Dimethylhexene als Hauptprodukt enthalten. Als weitere Verfahren sind der DIMERSOL-Prozess und der OCTOL-Prozess zu nennen. Der DIMERSOL-Prozess arbeitet mit löslichen Nickel-Komplexkatalysatoren und führt zu einem Octengemisch mit einem hohen Anteil an 3- und 5-Methylheptenen neben Dimethylhexenen und n-Octenen. Bei dem OCTOL-Prozess verwendet man geträgerte Nickel-Festbett-Katalysatoren und das erhaltene Octengemisch zeichnet sich durch einen geringen Verzweigungsgrad aus (DE 199 08 320 A1, WO 03/029180, Hydrocarbon Processing, February 1986, Seiten 31-33. Nach DE 199 08 320 A1 werden die jeweiligen, unterschiedlich verzweigten Octengemische über die Hydrocarboxylierung in die entsprechenden Isononansäuren überführt, die anschließend in die entsprechenden Vinylester umgewandelt werden. Vinylester aus Isononansäuren, die auf einem Octengemisch aus dem OCTOL-Prozess basieren, eignen sich als weichmachendes Comonomer.

Vor dem Hintergrund, dass die Verfügbarkeit an Octenen basierend auf dem C4-Schnitt aus der Naphthaspaltung beschränkt ist und von den lokalen Standortbedingungen abhängt, ist es wünschenswert, weitere Octenquellen auf Basis preiswert verfügbarer Großprodukte zu erschließen, die auf einfache Weise zu verschiedenen Standorten transportiert werden können. 2-Ethylhexanol steht als industrielles Großprodukt preiswert zur Verfügung, das ohne Probleme weitläufig vertrieben werden kann. 2-Ethylhexanol wird bekanntermaßen durch Hydroformylierung oder Oxo-Reaktion von Propylen zu n-Butyraldehyd mit nachfolgender alkalisch katalysierter Aldolkondensation zum 2-Ethylhexenal und anschließender Vollhydrierung zum 2-Ethylhexanol großtechnisch hergestellt (Ullmanns Encyklopädie der technischen Chemie, 4. Auflage, 1974, Verlag Chemie, Band 7, Seiten 214-215).

Auf die Verwendung von 2-Ethylhexanol zur Herstellung eines Octengemisches, das über die Dehydratisierung, Hydroformylierung und Hydrierung zu einem Isononanolgemisch verarbeitet wird, geht WO 03/029180 A1 kurz ein. Dabei steht die Einstellung der Viskosität der isomeren Phthalsäurealkylester im Mittelpunkt, die durch Veresterung von isomeren Nonanolen mit Phthalsäure oder Phthalsäureanhydrid erhalten werden. Hinweise, die Dehydratisierungsprodukte von 2-Ethylhexanol in Isononansäure zu überführen, werden nicht gegeben.

Die Nutzung von 2-Ethylhexanol als Octenquelle ermöglicht die Bereitstellung des Vinylesters der Isononansäure auf Basis von Propylen und mindert die Abhängigkeit von der Octenverfügbarkeit auf Butenbasis.

Die vorliegende Erfindung besteht daher in einem Verfahren zur Herstellung von einem Vinylester eines Gemisches von Isononansäuren ausgehend von 2-Ethylhexanol. Das Verfahren ist dadurch gekennzeichnet, dass man
(a) 2-Ethylhexanol in Gegenwart eines Katalysators zu einem Octengemisch dehydratisiert;
(b) das nach Schritt a) erhaltene Octengemisch in Gegenwart einer Übergangsmetallverbindung der Gruppe VIII des Periodensystems der Elemente mit Kohlenmonoxid und Wasserstoff zu einem Gemisch isomerer Isononanale umsetzt und anschließend zu einem Gemisch von Isononansäuren oxidiert, oder mit Kohlenmonoxid in Gegenwart von Wasser zu einem Gemisch von Isononansäuren umsetzt; und
(c) das nach Schritt b) erhaltene Gemisch von Isononansäuren zu dem entsprechenden Vinylester umsetzt durch Umsetzung mit Acetylen oder durch Umsetzung mit einem Vinylester einer anderen Carbonsäure.

Die Dehydratisierung von 2-Ethylhexanol kann sowohl in der Flüssigphase als auch in der Gasphase an einem dafür geeigneten Katalysator durchgeführt werden. Bevorzugt erfolgt die Dehydratisierung in der Gasphase bei Temperaturen im Bereich von 200 bis 450°C, vorzugsweise von 250 bis 380°C unter Verwendung fachüblicher Reaktoren in Gegenwart saurer heterogener Katalysatoren wie Aluminiumoxid in seinen verschiedenen Modifikationen, Nickel niedergeschlagen auf Aluminiumoxid, oder Phosphorsäure niedergeschlagen auf Siliziumdioxid oder Aluminiumoxid. Solche zur Dehydratisierung geeignete Heterogenkatalysatoren sind aus dem Stand der Technik her bekannt (GB 313426, US 2468764, US 2919973) und stehen beispielsweise als Al3996 der Firma BASF SE kommerziell zur Verfügung. US 2919973 behandelt die Dehydratisierung von 2-Ethylhexanol an einem heterogenen Aluminiumoxidkatalysator bei Temperaturen um 350°C und bei einer Katalysatorbelastung von 2,4 bis 2,8 Liter 2-Ethyl-hexanol pro Liter Katalysator und Stunde. Der Stand der Technik gibt jedoch keine Auskunft über die Isomerenverteilung in dem erhaltenen Octengemisch.

Der in dem erfindungsgemäßen Verfahren für die Dehydratisierung von 2-Ethylhexanol eingesetzte Reaktor kann neben der Katalysatorschüttung noch weitere Füllkörper oder Einbauten enthalten, beispielsweise Raschigringe, Sättel, Pallringe, Filterplatten oder Kolonnenböden. Verwendet man Füllkörper, dann werden sie vorzugsweise oberhalb der Katalysatorschüttung angebracht, um das Totvolumen zu verringern. Wird in der Flüssigphase dehydratisiert, kann auf Rührvorrichtungen, Einbauten und Füllkörper verzichtet werden, so dass in dem Reaktionsgefäß nur der Dehydratisierungskatalysator anwesend ist. In der bevorzugten Arbeitsweise wird 2-Ethylhexanol in einem vorgeschalteten Verdampfer erhitzt und gasförmig über die Katalysatorschüttung geführt, gegebenenfalls unter Verwendung eines inerten Trägergases wie Stickstoff, Kohlendioxid oder Edelgase. Die Belastung V/Vh des heterogenen Katalysators kann über einen weiten Bereich variieren und beträgt im Allgemeinen von 0,2 bis 3,5 Liter 2-Ethylhexanol pro Liter Katalysator und Stunde. Das der Dehydratisierungszone entnommene Reaktionsgemisch wird anschließend kondensiert. Durch das abgespaltene Wasser fällt eine wässrige Phase an, die von der organischen Olefinphase durch einfache Phasentrennung separiert wird. Bei dem erhaltenen Octen handelt es sich um ein Gemisch strukturisomerer Octene mit den einfach verzweigten Octenen 2-Ethyl-1-hexen sowie cis/trans 3-Methyl-3-hepten und cis/trans 3-Methyl-2-hepten als Hauptkomponenten. Nennenswerte Mengen an Di-C8-Ethern werden nicht gebildet.

Das nach Entfernen des Spaltwassers erhaltene Octen wird anschließend ohne weitere Reinigung oder zweckmäßigerweise nach destillativer Aufreinigung in die entsprechende Isononansäure überführt.

In einer Ausführungsform wird das erhaltene Octen mit Kohlenmonoxid und Wasserstoff in der Hydroformylierungsreaktion oder Oxo-Reaktion umgesetzt. Die eingesetzte Mischung aus Kohlenmonoxid und Wasserstoff bezeichnet man auch als Synthesegas.

Man führt die Hydroformylierungsreaktion in einem homogenen Reaktionssystem durch. Der Begriff homogenes Reaktionssystem steht für eine im Wesentlichen aus Lösungsmittel, falls zugesetzt, Katalysator, olefinisch ungesättigter Verbindung und Reaktionsprodukt zusammengesetzte homogene Lösung. Als besonders wirksame Lösungsmittel haben sich die höher siedenden Kondensationsverbindungen der herzustellenden Aldehyde, insbesondere die Trimeren der herzustellenden Aldehyde, erwiesen, die als Nebenprodukte bei der Hydroformylierung anfallen, sowie ihre Mischungen mit dem herzustellenden Isononanal, so dass ein weiterer Lösungsmittelzusatz nicht unbedingt erforderlich ist. In einigen Fällen kann sich jedoch ein Lösungsmittelzusatz als zweckmäßig erweisen. Als Lösungsmittel werden organische Verbindungen eingesetzt, in denen Ausgangsmaterial, Reaktionsprodukt und Katalysator löslich sind. Beispiele für solche Verbindungen sind aromatische Kohlenwasserstoffe, wie Benzol und Toluol oder die isomeren Xylole und Mesitylen. Andere gebräuchliche Lösungsmittel sind Paraffinöl, Cyclohexan, n-Hexan, n-Heptan oder n-Octan, Ether, wie Tetrahydrofuran, Ketone oder Texanol® der Firma Eastman. Der Anteil des Lösungsmittels im Reaktionsmedium kann über einen weiten Bereich variiert werden und beträgt üblicherweise zwischen 20 und 90 Gew.%, vorzugsweise 50 bis 80 Gew.-% bezogen auf das Reaktionsgemisch. Die Hydroformylierung des Octens kann aber auch ohne Lösungsmittelzusatz erfolgen.

Die Hydroformylierungsreaktion wird in homogener organischer Phase in Gegenwart mindestens einer Übergangsmetallverbindung der Gruppe VIII des Periodensystems der Elemente durchgeführt. Die Umsetzung kann sowohl in Gegenwart sowie in Abwesenheit von komplexbildenden Organoelementverbindungen, die als Komplexliganden wirken, durchgeführt werden.

Wird die Hydroformylierungsreaktion in Gegenwart von Komplexliganden durchgeführt, eignet sich die Verwendung von Organophosphorverbindungen als Organoelementverbindungen. Derartige Komplexverbindungen und ihre Herstellung sind bekannt (US 3 527 809 A, US 4 148 830 A, US 4 247 486 A, US 4 283 562 A). Sie können als einheitliche Komplexverbindungen oder auch als Gemisch unterschiedlicher Komplexverbindungen eingesetzt werden. Die Übergangsmetallkonzentration im Reaktionsmedium erstreckt sich über einen breiten Bereich von etwa 1 bis etwa 1000 Gew.-ppm und beträgt vorzugsweise 10 bis 700 Gew.-ppm und insbesondere 25 bis 500 Gew.-ppm, jeweils bezogen auf das homogene Reaktionsgemisch. Als Katalysator kann die stöchiometrisch zusammengesetzte Übergangsmetall-Komplexverbindung Anwendung finden. Es hat sich jedoch als zweckmäßig erwiesen, die Hydroformylierung in Gegenwart eines Katalysatorsystems aus Übergangsmetall-Komplexverbindung und freiem Komplexliganden durchzuführen, der mit dem Übergangsmetall keine Komplexverbindung mehr eingeht. Der freie Komplexligand kann der Gleiche sein wie in der Übergangsmetall-Komplexverbindung, es können aber auch von diesem verschiedene Komplexliganden eingesetzt werden. Zu den bevorzugten Komplexliganden zählen Triarylphosphine wie Triphenylphosphin, Trialkylphosphine wie Tri(cyclohexyl)phosphin, Alkylphenylphosphine, organische Phosphite oder Diphosphite. Das molare Verhältnis von Übergangsmetall zu Komplexligand beträgt im Allgemeinen 1:1 bis 1:1000, es kann aber auch noch höher liegen. Bevorzugt setzt man das Übergangsmetall und den Komplexliganden in einem molaren Verhältnis von 1:3 bis 1:500 und insbesondere von 1:50 bis 1:300 ein.

Die Hydroformylierungsreaktion in Gegenwart von Komplexliganden bezeichnet man häufig auch als modifizierte Variante, die üblicherweise bei Temperaturen von 50 bis 180°C, vorzugsweise von 100 bis 160°C und Gesamtdrücken von 0,2 bis 30 MPa, vorzugsweise 1 bis 20 MPa, durchgeführt wird.

Die Hydroformylierungsreaktion kann ebenfalls in Abwesenheit von Komplexliganden nach der unmodifizierten Variante durchgeführt werden. Solche, beispielsweise nicht mit Phosphinen oder Phosphiten modifizierte Übergangsmetallkatalysatoren und ihre Eignung als Katalysator zur Hydroformylierung sind aus der Literatur her bekannt und sie werden als unmodifizierte Übergangsmetallkatalysatoren bezeichnet. Es wird in der Fachliteratur angenommen, dass die Übergangsmetallverbindung HM(CO)₄ die katalytisch aktive Übergangsmetallspezies bei der unmodifizierten Übergangsmetallkatalyse ist, obgleich dies aufgrund der vielen in der Reaktionszone nebeneinander ablaufenden Chemismen nicht eindeutig bewiesen ist.

Vorzugsweise verwendet man als Übergangsmetalle der Gruppe VIII des Periodensystems der Elemente Kobalt, Rhodium, Iridium, Nickel, Palladium, Platin, Eisen oder Ruthenium und insbesondere Kobalt oder Rhodium. Der modifizierte oder unmodifizierte Übergangsmetallkatalysator bildet sich unter den Bedingungen der Hydroformylierungsreaktion aus den eingesetzten Übergangsmetallverbindungen, wie deren Salzen, wie Chloriden, Nitraten, Sulfaten, Acetaten, Pentanoaten, 2-Ethylhexanoaten oder Isononanoaten, deren Chalkogeniden, wie Oxiden oder Sulfiden, deren Carbonylverbindungen, wie M₂(CO)₈, M₄(CO)₁₂, M₆(CO)₁₆, M₂(CO)₉, M₃(CO)₁₂, deren Organoübergangsmetallverbindungen, wie Carbonylacetylacetonaten oder Cyclooctadienylacetaten oder -chloriden, in Gegenwart von Kohlenmonoxid/Wasserstoffgemischen. Dabei kann die Übergangsmetallverbindung als Feststoff oder zweckmäßigerweise in Lösung eingesetzt werden. Als Übergangsmetallverbindung, die als Katalysatorvorstufe verwendet wird, eignet sich insbesondere Rhodiumisononanoat, Rhodiumacetat, Rhodium-2-ethylhexanoat oder Kobaltisononanoat, Kobaltacetat oder Kobalt-2-ethylhexanoat, oder Co₂(CO)₈, Co₄(CO)₁₂, Rh₂(CO)₈, Rh₄(CO)₁₂ oder Rh₆(CO)₁₆ oder Cyclopentadienylrhodiumverbindungen, Rhodiumacetylacetonat oder Rhodiumdicarbonylacetylacetonat. Bevorzugt werden Rhodiumoxid und insbesondere Rhodiumacetat, Rhodium-2-ethylhexanoat und Rhodiumisononanoat eingesetzt.

Es ist aber auch möglich, den Übergangsmetallkatalysator in einer Vorcarbonylierungsstufe zunächst zu präformieren und ihn anschließend der eigentlichen Hydroformylierungsstufe zuzuführen. Die Bedingungen der Präformierung entsprechen dabei im Allgemeinen den Hydroformylierungsbedingungen.

Da im Allgemeinen die Verwendung von nicht mit Komplexliganden modifizierten Übergangsmetallkatalysatoren einen geringeren Übergangsmetallgehalt erfordert, arbeitet man im Allgemeinen mit einer Übergangsmetallmenge von 1 bis 100 ppm, vorzugsweise 2 bis 30 ppm, bezogen auf das eingesetzte Octen. Ganz besonders wird Rhodium oder Kobalt in einer Menge von 2 bis 30 ppm, vorzugsweise von 5 bis 10 ppm, jeweils bezogen auf das eingesetzte Octen, verwendet.

Bei der Umsetzung des Octens mit Wasserstoff und Kohlenmonoxid zu Isononanal nach der unmodifizierten Variante arbeitet man zweckmäßigerweise bei höheren Drücken im Bereich von 5 bis 70 MPa, vorzugsweise von 5 bis 60 MPa und insbesondere von 10 bis 30 MPa. Geeignete Reaktionstemperaturen bewegen sich im Bereich von 50 bis 180°C, bevorzugt von 50 bis 150°C und insbesondere 100 bis 150°C.

Die Zusammensetzung des Synthesegases, d. h. die Anteile von Kohlenmonoxid und Wasserstoff im Gasgemisch, kann in weiten Grenzen variiert werden. Im Allgemeinen setzt man Gemische ein, in denen das Molverhältnis von Kohlenmonoxid zu Wasserstoff 5 : 1 bis 1 : 5 beträgt. Üblicherweise ist dieses Verhältnis 1 : 1 oder weicht von diesem Wert nur wenig ab. Die olefinische Verbindung kann als solche oder in Lösung der Reaktionszone zugeführt werden. Geeignete Lösungsmittel sind Ketone wie Aceton, Methylethylketon, Acetophenon, niedrigere aliphatische Nitrile wie Acetonitril, Propionitril oder Benzonitril, Dimethylformamid, lineare oder verzweigte gesättigte aliphatische Monohydroxyverbindungen wie Methanol, Ethanol, Propanol und Isopropanol, aromatische Kohlenwasserstoffe wie Benzol oder Toluol und gesättigte cycloaliphatische Kohlenwasserstoffe wie Cyclopentan oder Cyclohexan.

Die Hydroformylierungsstufe kann sowohl diskontinuierlich als auch kontinuierlich durchgeführt werden. Die Gewinnung der gewünschten Aldehyde aus dem rohen Hydroformylierungsprodukt erfolgt nach konventionellen Verfahren, beispielsweise durch Destillation. Isononanal und weitere flüchtige Komponenten werden als Kopfprodukte abgezogen und, bei Bedarf, einer weiteren Feinreinigung unterzogen.

Die eingesetzten Übergangsmetallmengen fallen im Destillationsrückstand an und werden gegebenenfalls nach Zusatz von frischer Übergangsmetallverbindung und Entnahme eines Teils der im Verlauf der Reaktion gebildeten Aldehydkondensationsprodukte in die Reaktionszone zurückgeführt.

Das erhaltene Gemisch isomerer Isononanale wird aufgereinigt, zweckmäßigerweise durch Destillation, und anschließend durch Oxidation in die entsprechende Isononansäure überführt, vorzugsweise durch die Oxidation in der Flüssigphase, obwohl andere Verfahrensausgestaltungen wie die Oxidation in der Gasphase nicht ausgeschlossen sind. Als Oxidationsmittel eignen sich übliche, zur Oxidation von aliphatischen Aldehyden geeignete Verbindungen wie Sauerstoff, sauerstoffenthaltende Gasgemische, Ozon, ozonhaltige Gasgemische, Peroxide, Persäuren, Metallsalze von Persäuren oder Übergangsmetalle in hohen Oxidationsstufen, beispielsweise Kaliumpermanganat oder Braunstein. Aufgrund der guten Verfügbarkeit verwendet man als Oxidationsmittel zweckmäßig molekularen Sauerstoff oder Gasgemische, die molekularen Sauerstoff enthalten. Weitere Bestandteile derartiger Gasgemische sind inerte Gase, z. B. Stickstoff, Edelgase und Kohlendioxid. Der Anteil der inerten Bestandteile des sauerstoffenthaltenden Gasgemisches beträgt bis zu 90 Vol-%, insbesondere 30 bis 80 Vol-%. Die bevorzugten Oxidationsmittel sind Sauerstoff oder Luft.

Die Oxidation kann entweder unter Katalysatorzusatz oder in Abwesenheit von Katalysatoren durchgeführt werden. Als Katalysatoren eignen sich Übergangsmetalle oder Verbindungen von Übergangsmetallen, die in geringen Mengen wie beispielsweise von 0,1 bis 5 ppm, berechnet als Übergangsmetall und bezogen auf eingesetzten Aldehyd, zugesetzt werden können wie Titan, Vanadium, Chrom, Molybdän, Mangan, Eisen, Kobalt, Nickel, Ruthenium, Rhodium, Palladium oder Kupfer. Eine solche Verfahrensführung wird beispielsweise in DE 100 10 771 C1 oder DE 26 04 545 A1 beschrieben.

Ebenfalls kann die Umsetzung in Anwesenheit von Alkali- oder Erdalkalimetallsalzen schwacher Säuren durchgeführt werden. Insbesondere bei der Oxidation von α-verzweigten Aldehyden, bei denen das dem Carbonylkohlenstoffatom benachbarte Kohlenstoffatom die Verzweigung trägt, empfiehlt der Stand der Technik die Anwesenheit von geringen Mengen an Alkalimetallcarboxylaten zur Selektivitätsverbesserung (DE 950 007, DE 100 10 771 C1). Auch kann eine Kombination von Alkali- oder Erdalkalimetallcarboxylaten mit Übergangsmetallverbindungen, wie in EP 1 854 778 A1 behandelt, verwendet werden.

Bei der Oxidation des Isononanals, das nach dem erfindungsgemäßen Verfahren ausgehend von 2-Ethylhexanol über die Dehydratisierung und Hydroformylierung des entsprechenden Octens hergestellt wird, ist die Anwesenheit von Alkali- oder Erdalkalimetallcarboxylaten empfehlenswert, im Allgemeinen in einer Menge von 1 bis 30 mmol, vorzugsweise von 1 bis 15 mmol und insbesondere von 1 bis 8 mmol, je Mol Aldehyd, berechnet als Alkali- oder Erdalkalimetall.

Es ist nicht erforderlich, die Alkali- oder Erdalkalimetallcarboxylate als einheitliche Verbindung einzusetzen. Es ist ebenfalls möglich, Gemische dieser Verbindungen zu verwenden, wobei man jedoch zweckmäßigerweise Isononanoate verwendet. Vorzugsweise setzt man jedoch einheitliche Verbindungen ein, beispielsweise Lithium-, Kalium-, Natrium-, Calcium- oder Bariumisononanoat.

Im Allgemeinen stellt man eine Alkali- oder Erdalkalimetallisononanoate enthaltende Lösung durch Neutralisation einer wässrigen, die Alkali- oder Erdalkalimetallverbindung enthaltenden Lösung mit einem Überschuss an Isononansäure her und setzt diese Lösung dem zu oxidierenden Isononanal zu. Als Alkali- oder Erdalkalimetallverbindungen eignen sich besonders die Hydroxide, Carbonate oder Hydrogencarbonate.

Es ist aber auch möglich, die Alkali- oder Erdalkalimetallisononanoate im Reaktionsgemisch zu erzeugen, indem man Alkali- oder Erdalkalimetallverbindungen zusetzt, die unter den Reaktionsbedingungen in die Isononanoate überführt werden. Beispielsweise lassen sich Alkali- oder Erdalkalimetallhydroxide, -carbonate, -hydrogencarbonate oder -oxide in der Oxidationsstufe einsetzen. Ihr Zusatz kann entweder in fester Form oder als wässrige Lösung erfolgen.

Die Umsetzung mit dem Oxidationsmittel, vorzugsweise mit Sauerstoff oder sauerstoffenthaltenden Gasen, wird in einem Temperaturbereich von 20 bis 100°C durchgeführt. Vorzugsweise arbeitet man zwischen 20 und 80°C, insbesondere zwischen 40 und 80°C. Die Temperaturführung, konstante oder variable Temperatur, kann den individuellen Erfordernissen des Ausgangsmaterials und den Reaktionsgegebenheiten angepasst werden.

Die Umsetzung der Reaktionspartner erfolgt bevorzugt bei Atmosphärendruck. Die Anwendung erhöhten Drucks ist jedoch nicht ausgeschlossen. Üblicherweise arbeitet man in einem Bereich von Atmosphärendruck bis 1,5 MPa, vorzugsweise bei Atmosphärendruck bis 0,8 MPa.

Die zur Umwandlung des Isononanals in die entsprechende Isononansäure erforderliche Reaktionszeit hängt unter anderem ab von der Reaktionstemperatur und dem Mengenverhältnis der Reaktanten zueinander. Normalerweise beträgt sie 30 Minuten bis 20 Stunden, insbesondere 2 bis 8 Stunden. Isononanal kann als solches oder gelöst in einem, unter den Reaktionsbedingungen inerten, Lösungsmittel eingesetzt werden. Beispiele für geeignete Lösungsmittel sind Ketone wie Aceton, Ester, z. B. Ethylacetat, Kohlenwasserstoffe, z. B. Toluol und Nitrokohlenwasserstoffe wie Nitrobenzol. Die Konzentration des Aldehyds wird durch seine Löslichkeit in dem Lösungsmittel begrenzt.

Der Oxidationsschritt kann absatzweise oder kontinuierlich durchgeführt werden. Eine Rückführung nicht umgesetzter Reaktionsteilnehmer ist in beiden Fällen möglich.

Bei der erhaltenen Isononansäure ausgehend von 2-Ethylhexanol mit nachfolgender Hydroformylierung und Oxidation handelt es sich um ein Gemisch stellungsisomerer aliphatischer C9-Monocarbonsäuren mit in α-Position unverzweigten und einfachverzweigten Isononansäuren als Hauptkomponenten.

Nach der gaschromatographischen Analyse gemäß DIN 51405 (FI.-%) liegen als Hauptkomponenten 4-Methyloctansäure, 6-Methyloctansäure, 2,5-Dimethylheptansäure, 2,3-Dimethylheptansäure, 3-Ethylheptansäure, 2-Ethylheptansäure und 2-Ethyl-4-methylhexansäure vor sowie geringe Mengen an 2-Propyl-3-methyl-pentansäure und 2-Methyloctansäure. Geringe Mengen an n-Nonansäure sind ebenfalls zugegen.

Die nach dem erfindungsgemäßen Verfahren über die Hydroformylierungs- und Oxidationsreaktion hergestellte Isononansäure ist dadurch charakterisiert, dass die Hauptkomponenten 4-Methyloctansäure, 6-Methyloctansäure, 2,5-Dimethylheptansäure, 2,3-Dimethylheptansäure, 3-Ethylheptansäure, 2-Ethylheptansäure und 2-Ethyl-4-methylhexansäure in Summe mindestens 80 mol-%, bezogen auf den Gesamtgehalt an stellungsisomeren aliphatischen C9-Monocarbonsäuren, ausmachen.

Aus dem nach der Oxidation anfallenden Rohsäuregemisch wird mittels Destillation unter üblichen Bedingungen die reine isononansäure gewonnen. Der die Alkali- oder Erdalkalimetallisononanoate und gegebenenfalls Übergangsmetalle enthaltende Destillationsrückstand wird abgetrennt und kann dem Einsatzaldehyd, gegebenenfalls nach Zugabe von frischen Alkali- oder Erdalkalimetallisononanoaten oder Alkali- oder Erdalkalimetallverbindungen, die unter den Reaktionsbedingungen in die Isononanoate übergehen, sowie gegebenenfalls von frischen Übergangsmetallverbindungen wieder zugeführt werden.

Nach einer bewährten Ausführungsform des erfindungsgemäßen Verfahrens legt man Isononanal in einem geeigneten Reaktor, z. B. in einem mit einem Anströmboden versehenen Rohrreaktor, der gegebenenfalls noch Füllkörper enthält, vor und leitet den Sauerstoff oder das sauerstoffenthaltende Gasgemisch von unten durch den Aldehyd.

Entsprechend einer weiteren Ausführungsform verwendet man als Reaktor einen Rieselturm, der Füllkörper enthält. Über die Füllung lässt man den Aldehyd herabrieseln und leitet in den Turm gleichzeitig im Gleich- oder Gegenstrom Sauerstoff oder ein sauerstoffenthaltendes Gasgemisch ein.

Ebenfalls kann das durch Dehydratisierung von 2-Ethylhexanol erhaltene Octen durch die Hydrocarboxylierung mit Kohlenmonoxid und Wasser nach der sogenannten Koch-Reaktion in ein Gemisch hochverzweigter Isononansäuren überführt werden (Weissermel, Arpe, Industrielle Organische Chemie, VCH Verlagsgesellschaft, 3. Auflage, 1988, Seiten 150-152; J. Falbe, Carbon Monoxide in Organic Synthesis, Springer Verlag Berlin, Heidelberg, New York, 1970, Seiten 127-135). Die Umsetzung wird in Gegenwart von stark sauren Protonen-Katalysatoren, wie Schwefelsäure, Fluorwasserstoffsäure oder Phosphorsäure durchgeführt, häufig auch im Gemisch mit Lewissäuren wie Bortrifluorid oder Antimonpentafluorid. Ein geeignetes Katalysatorsystem für die Hydrocarboxylierung von Olefinen ist ein Gemisch aus Bortrifluorid und Phosphorsäure im Molverhältnis 1,5 zu 1 und aus WO 93/22270 A1 bekannt. Nach EP 1 281 700 A1 kann sich dieses Molverhältnis über einen Bereich von 0,5 zu 1 bis 5,0 zu 1 erstrecken. Die Umsetzung des Octens erfolgt im Allgemeinen bei Temperaturen zwischen 60 und 140°C und bei einem Kohlenmonoxiddruck von 5 bis 12 MPa in Gegenwart von Wasser in einer Menge von 8 bis 30 Gew.-%, bezogen auf die eingesetzte Katalysatormenge.

Nach erfolgter Reaktion wird die organische Phase von der wässrigen Phase getrennt und durch Wasserwäsche gereinigt. Nach mechanistischen Vorstellungen bildet sich unter den stark sauren Reaktionsbedingungen unter Protonenanlagerung zunächst ein tertiäres Carbeniumion in dem Olefingerüst aus, an das Kohlenmonoxid unter Bildung eines Acyliumkations addiert, das anschließend mit Wasser abgesättigt wird. Durch diese Reaktionsfolge bildet sich bei der Hydrocarboxylierung des Octens eine Isononansäure mit tertiären Strukturisomeren, die an dem der Carboxylgruppe benachbarten Kohlenstoffatom zwei α,α-Alkylreste tragen und eine sogenannte Neo-Struktur besitzen.

Anschließend wird die erfindungsgemäß hergestellte Isononansäure ausgehend von 2-Ethylhexanol zu dem entsprechenden Vinylester umgesetzt. Dies kann beispielsweise durch Umsetzung der Isononansäure mit Acetylen, vorzugsweise in Gegenwart von Zinksalzen, bei Temperaturen von 200-230°C erfolgen (G. Hübner, Fette, Seifen, Anstrichmittel 68, 290 (1966), Ullmanns Encyklopädie der technischen Chemie, 4. Auflage, 1983, Verlag Chemie, Band 23, Seiten 606-607; EP 1 057 525 A2).
Es ist ebenfalls möglich, die so erhaltene Isononansäure einer sogenannten Transvinylierungsreaktion mit einem Vinylester einer anderen Carbonsäure zu unterziehen: 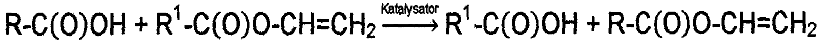 wobei R gleich C8 ist und R¹ häufig Methyl oder Ethyl bedeutet, so dass als Transvinylierungsreagenz beispielsweise Vinylacetat oder Vinylpropionat verwendet wird (Ullmanns Encyklopädie der technischen Chemie, 4. Auflage, 1983, Verlag Chemie, Band 23, Seiten 606-607). Um das chemische Gleichgewicht in Richtung des gewünschten Vinylesters zu drängen, verwendet man häufig einen Überschuss an dem Transvinylierungsreagenz R¹-C(O)O-CH=CH₂ und entfernt gleichzeitig die gebildete Carbonsäure aus dem Reaktionsgemisch. Die kontinuierliche oder semi-kontinuierliche Verfahrensführung kann beispielsweise als Reaktivdestillation ausgestaltet werden (EP 0 497 340 A2) oder als Blasensäule mit aufgesetzter Säule, der noch zusätzlich eine Rektifikationssäule und eine Stripp-Säule nachgeschaltet sind (WO 2011/139360 A1 und WO 2011/139361 A1).

Es ist aber auch möglich, die Transvinylierungsreaktion ohne Entzug eines Reaktionspartners kontinuierlich durchzuführen und das erhaltene Reaktionsgemisch in einem separaten Aufarbeitungsteil in die einzelnen Komponenten aufzuspalten, eine Vorgehensweise, die in der noch nicht veröffentlichten deutschen Patentanmeldung mit dem Aktenzeichen DE 10 2012 002282.4 behandelt wird. Bei dieser Verfahrensweise kann die Transvinylierungsreaktion sowohl bei Normaldruck als auch bei erhöhtem Druck durchgeführt werden, im Allgemeinen bis zu einem Druck von 15 MPa, vorzugsweise von 0,5 bis 8 MPa und insbesondere von 0,8 bis 2 MPa. Als ganz besonders geeignet haben sich Reaktionseinstellungen bei einer Temperatur von 90 bis 140°C und bei einem Druck von 0,8 bis 2 MPa erwiesen. Aber auch bei Normaldruck und insbesondere bei einer Reaktionstemperatur von 60 bis 150°C werden ebenfalls sehr hohe Raum-Zeit-Ausbeuten an dem gewünschten Vinylester der Isononansäure erzielt.

Als Reaktionsgefäß eignet sich ein Rohrreaktor, wie ein beliebig angeordnetes Strömungsrohr, zum Beispiel ein senkrecht stehendes oder ein waagerecht angeordnetes Strömungsrohr oder ein mehrfach geschlängeltes Strömungsrohr. Der Rohrreaktor kann als Leerrohr betrieben werden, er kann jedoch ebenfalls Füllkörper oder Einbauten enthalten, beispielsweise Raschigringe, Sättel, Pallringe, Wendel, Strombrecher oder statische Mischer oder Mischerpackungen. Statische Mischelemente stehen kommerziell zur Verfügung und werden beispielsweise als Sulzermischer oder Kenicksmischer angeboten mit speziellen Produktlinien für das Vermischen unterschiedlich viskoser Flüssigkeiten. Der Rohrreaktor kann ebenfalls mit einem Umpump und gegebenenfalls mit einem Wärmetauscher versehen werden.

Ebenfalls kann die Transvinylierungsreaktion kontinuierlich in einem Rührkessel oder in einer Rührkesselkaskade drucklos oder unter Druck durchgeführt werden. Die Isononansäure und das Transvinylierungsreagenz R¹-C(O)O-CH = CH₂ werden entweder getrennt oder als Gemisch kontinuierlich eingespeist und das im stationären Zustand befindliche Reaktionsgemisch wird kontinuierlich abgeführt. Ebenfalls ist die kontinuierliche Reaktionsführung in fachüblichen Reaktorausführungen, wie in einem Schlaufenreaktor unter Nutzung der Wärmekonvektion oder in einem Mehrkammerreaktor möglich. Das Reaktionsgefäß kann ebenfalls als zylindrischer Reaktor mit einer axial angeordneten Düse für den Einlass des flüssigen, katalysatorhaltigen Gemisches aus der Isononansäure und dem Transvinylierungsreagenz R¹-C(O)O-CH = CH₂ ausgestaltet werden, der zusätzlich noch zur Erzeugung einer internen Zwangsströmung ein axial angeordnetes Leitrohr enthält.

Es hat sich eine Belastung des Reaktionsgefäßes V/Vh mit einem zuvor hergestellten Gemisch aus dem Transvinylierungsreagenz und der Isononansäure von 0,4 bis 7,0 h⁻¹, vorzugsweise von 0,7 bis 6,2 h⁻¹, bezogen auf das Reaktorvolumen und Zeit, als vorteilhaft erwiesen. Werden beide Edukte getrennt aber gleichzeitig in das Reaktionsgefäß eingeleitet, beträgt die Belastung V/Vh des Reaktionsgefäßes mit dem Transvinylierungsreagenz von 0,02 bis 6,0 h⁻¹ und mit der Isononansäure von 0,1 bis 6,7 h⁻¹, jeweils bezogen auf das Reaktorvolumen und Zeit.

Es ist ebenfalls möglich, die Transvinylierungsreaktion in einem geschlossenen Reaktionsgefäß absatzweise durchzuführen. Dabei hat sich eine Verfahrensführung bei Temperaturen von 60 bis 150°C und bei Drücken von Eigendruck bis 5 MPa als zweckmäßig erwiesen. Der gewünschte Druck kann beispielsweise durch Aufpressen eines Inertgases wie Stickstoff oder Edelgase eingestellt werden.

Als Transvinylierungsreagenz R¹C(O)O-CH = CH₂ verwendet man vorzugsweise Vinylacetat mit R¹ gleich Methyl oder Vinylpropionat mit R¹ gleich Ethyl. Aber auch Vinylester höherer Carbonsäuren wie Vinyllaurat mit R¹ gleich Undecyl können eingesetzt werden, wenn ihre Verwendung bei der nachfolgenden Aufarbeitung des Reaktionsgemisches als zweckmäßig erscheint. Üblicherweise wird das erhaltene Reaktionsgemisch durch Destillation aufgereinigt.

Bezogen auf Isononansäureeinsatz kann das Transvinylierungsreagenz R¹-C(O)O- CH = CH₂ in einem molaren Verhältnis von 0,1 zu 1 bis 10 zu 1, vorzugsweise von 0,2 zu 1 bis 5 zu 1, eingesetzt werden. Seine Menge richtet sich ebenfalls nach den physikalischen Eigenschaften der Einsatzstoffe und den gebildeten Reaktionspartnern und danach, auf welche Weise das Reaktionsgemisch möglichst vorteilhaft aufgearbeitet werden kann.

Vinylacetat hat sich aufgrund der kostengünstigen Verfügbarkeit, seines Siedepunktes und des Siedepunktes der in der Transvinylierungsreaktion gebildeten Essigsäure als vorteilhaftes Transvinylierungsreagenz erwiesen. Das Reaktionsgemisch wird üblicherweise destillativ aufgearbeitet und überschüssiges Vinylacetat, gebildete Essigsäure und der gewünschte Vinylester der Isononansäure werden als flüchtige Komponenten abgezogen und weiter aufgetrennt. Im Rückstand verbleibt Isononansäure zusammen mit dem Transvinylierungskatalysator. Der katalysatorhaltige Rückstand wird nach optionalem Ausschleusen eines hochsiederhaltigen Teilstromes wieder in die Transvinylierungsreaktion zurückgeführt, gegebenenfalls nach Zugabe von Frischkatalysator oder frischen Liganden.

Es ist ebenfalls möglich, Vinylacetat im molaren Unterschuss bis zu 0,1 zu 1, vorzugsweise bis zu 0,2 zu 1, bezogen auf den molaren Isononansäureeinsatz zu verwenden. Dadurch kann der Aufwand zur Abtrennung des Vinylacetats verringert werden.

Die bei der Transvinylierungsreaktion mit Vinylacetat freigesetzte Essigsäure kann nach Aufreinigung für nachfolgende Derivatisierungsreaktionen verwendet werden, beispielsweise zur Herstellung von Vinylacetat durch Umsetzung mit Ethylen und Sauerstoff an festen, palladiumhaltigen Trägerkatalysatoren (Weissermel, Arpe, Industrielle Organische Chemie, VCH Verlagsgesellschaft, 3. Auflage, 1988, Seiten 244-247).

Die Veresterung der gewonnenen Essigsäure mit niedrigen aliphatischen Alkoholen wie Methanol, Ethanol, n-Propanol, iso-Propanol, n-Butanol oder iso-Butanol ergibt die entsprechenden Acetatester wie n-Propylacetat, isoPropylacetat, n-Butylacetat oder iso-Butylacetat. Die Veresterung von Essigsäure mit niedrigen aliphatischen Alkoholen ist an sich bekannt (Ullmanns Encyklopädie der technischen Chemie, 4. Auflage, 1983, Verlag Chemie GmbH, Band 11, Seiten 68-70; Band 19, Seiten 457-458). Weitere beispielhafte Derivate, die nach an sich bekannten Verfahren aus der freigesetzten Essigsäure hergestellt werden können, sind Acetylhalogenide, Amide, Carbonsäureanhydride, Chloressigsäuren oder Ethanol.

Als Transvinylierungskatalysatoren eignen sich Verbindungen der Übergangsmetalle aus der Platingruppe Ruthenium, Osmium, Rhodium, Iridium, Palladium und Platin, insbesondere Palladium und Ruthenium, die modifiziert mit ein- oder mehrzähnigen Organostickstoff- oder Organophosphorliganden oder in unmodifizierter Form eingesetzt werden können. Die Gesamtkonzentration des Übergangsmetalls oder der Übergangsmetalle, wenn eine Mischung davon verwendet wird, beträgt im Allgemeinen von 0,005 bis 1 mol-%, vorzugsweise von 0,01 bis 0,5 und insbesondere von 0,02 bis 0,25 mol-%, jeweils bezogen auf die im Unterschuss eingesetzte Ausgangsverbindung. Rutheniumverbindungen werden beispielsweise unmodifiziert eingesetzt und Palladiumverbindungen häufig modifiziert mit 2,2'-Bipyridyl oder 1,10-Phenanthrolin oder unmodifiziert zusammen mit einer Alkalimetallverbindung, wie Lithiumacetat, und einem redoxaktiven Übergangsmetall, beispielsweise zweiwertigem Kupfer in Form von Halogeniden.

Das erhaltene Vinylisononanoat kann in Copolymeren verwenden werden. Es eignet sich als Comonomer in Polyvinylacetat, Polyvinylchlorid, Polystyrol oder Polyacrylsäureestern, welche die Hydrolysebeständigkeit und Feuchtigkeitsaufnahme von Anstrichen vorteilhaft beeinflussen.

In den folgenden Beispielen wird die Herstellung von Isononansäure ausgehend von 2-Ethylhexanol sowie ihre Umsetzung zu dem Vinylester beschrieben.

### Beispiele

### I. Dehydratisierung von 2-Ethylhexanol

Zur Dehydratisierung wurde ein Quarzrohr mit einer Länge von 1,3 Meter und einem Durchmesser von 0,03 Meter verwendet, bei dem sich die beheizte Zone über 1,1 Meter erstreckte. Das Quarzrohr wurde mit 250 ml des sauren Katalysators Al 3996 der Firma BASF SE in Form von 3x3 Millimeter großen Tabletten bestückt. Das Totvolumen wurde mit Glasringen aufgefüllt. 2-Ethylhexanol wurde in einem vorgeschalteten Verdampfer verdampft und mit Hilfe eines Stickstoffstromes als Trägergas bei Normaldruck über die Katalysatorschüttung bei einer Temperatur von 350°C und mit einer Belastung von 0,5 Liter je Liter Katalysatorvolumen und Stunde gefahren. Das erhaltene Reaktionsgemisch wurde in einem nachgeschalteten Auffanggefäß kondensiert und die wässrige Phase wurde abgetrennt. Die angefallene organische Phase wies folgende gaschromatographisch ermittelte Zusammensetzung auf (FI.-%, gemäß DIN 51405):

| | |
|---|---|
| Vorlauf/C4-C7 -Kohlenwasserstoffe | 0,3 |
| andere C8-Olefine | 9,6 |
| 2-Ethyl-1-hexen | 7,6 |
| cis-3-Methyl-3-hepten | 14,6 |
| trans-3-Methyl-3-hepten | 28,8 |
| cis-3-Methyl-2-hepten | 16,2 |
| trans-3-Methyl-2-hepten | 23,9 |
| n-Octene | 0,8 |
| Nachlauf | 0,1 |

### II. Hydroformylierung des gemäß Schritt I. erhaltenen Octens

Das nach Schritt I erhaltene rohe Octen wurde in Gegenwart von 5 ppm Rhodium, zugegeben in Form einer Lösung von Rhodium-2-ethylhexanoat in 2-Ethylhexanol und bezogen auf Octeneinsatz, bei einer Temperatur von 140°C und bei einem Synthesegasdruck von 19 MPa über einen Zeitraum von drei Stunden hydroformyliert. Die molare Zusammensetzung des Synthesegases betrug 1 Mol Wasserstoff zu 1 Mol Kohlenmonoxid. Das erhaltene rohe Hydroformylierungsprodukt wies folgende gaschromatographisch ermittelte Zusammensetzung (FI.-%, gemäß DIN 51405) auf:

| | |
|---|---|
| Vorlauf | 0,1 |
| C8-Kohlenwasserstoffe | 8,5 |
| Zwischenlauf | 0,2 |
| Isononanal | 88,1 |
| n-Nonanal | 1,4 |
| Nachlauf | 1,7 |

Die Ergebnisse weiterer Hydroformylierungsversuche mit einem über die Dehydratisierung von 2-Ethylhexanol erhaltenen Octen sind in der nachfolgenden Tabelle 1 zusammengestellt. Vor Einsatz wurde das rohe Octen an einer Claisenbrücke zur Nachlaufabtrennung bei einer Kopftemperatur von 119-122°C und bei Normaldruck destilliert. Die Einsatzoctene sowie die erhaltenen Reaktionsprodukte wurden gaschromatographisch analysiert (Angabe in FI.-%, gemäß DIN 51405).

**Tabelle 1: Hydroformylierung von Octenen, erhalten durch die 2-Ethylhexanoldehydratisierung**

| **Beispiel** | **IIa** | **IIb** |
|---|---|---|
| Einsatz Edukt | destilliert | destilliert |

| **GC-Analyse Edukt (%)** | | |
|---|---|---|
| Vorlauf/C4-C7-Kohlenwasserstoffe | 0,3 | 0,4 |
| andere C8-Olefine | 5,9 | 7,7 |
| 2-Ethyl-1-hexen | 9,3 | 9,2 |
| cis-3-Methyl-3-hepten | 15,2 | 15,0 |
| trans-3-Methyl-3-hepten | 27,4 | 27,1 |
| cis-3-Methyl-2-hepten | 16,1 | 15,6 |
| trans-3-Methyl-2-hepten | 25,2 | 24,7 |
| n-Octene | 0,5 | 0,2 |
| Nachlauf | 0,1 | 0,1 |

| **Versuchsbedingungen** | | |
|---|---|---|
| Rh-Konzentration [ppm], bezogen auf Octeneinsatz | 20 | 10 |
| Druck [MPa] | 19 | 27 |
| Temperatur [°C] | 140 | 140 |
| Reaktionszeit [h] | 2 | 2 |

| **GC-Analyse Produkt (%)** | | |
|---|---|---|
| Vorlauf | 0,1 | 0,1 |
| C8-Kohlenwasserstoffe | 2,5 | 1,1 |
| Zwischenlauf | 0,3 | 0,1 |
| iso-Nonanale | 90,8 | 94,7 |
| n-Nonanal | 2,0 | 1,4 |
| Nachlauf | 4,3 | 2,6 |

Die unter Verwendung von Triphenylphosphin als Komplexligand durchgeführten Hydroformylierungsversuche mit dem über die Dehydratisierung von 2-Ethylhexanol erhaltenen Octen sind in der nachfolgenden Tabelle 2 zusammengestellt. Es kam undestillierte Ware zum Einsatz. Die Einsatzoctene sowie die erhaltenen Reaktionsprodukte wurden gaschromatographisch analysiert (Angaben in FI.-%, gemäß DIN 51405).

**Tabelle 2: Hydroformylierung von Octenen, erhalten durch die 2-Ethylhexanoldehydratisierung, Zusatz von Triphenylphosphin**

| **Beispiel** | **IIc** | **IId** | **IIe** | **IIf** |
|---|---|---|---|---|
| Einsatz Edukt | undestilliert, roh | undestilliert, roh | undestilliert, roh | undestilliert, roh |
| **GC-Analyse Edukt (%)** | | | | |
| C4-C7 Kohlenwasserstoffe | 0,3 | 0,3 | 0,3 | 0,4 |
| Andere C8-Olefine | 19,1 | 19,1 | 19,1 | 11,6 |
| 2-Ethyl-1-hexen | 7,9 | 7,9 | 7,9 | 8,6 |
| 3-Methyl-3-hepten | 36,5 | 36,5 | 36,5 | 40,0 |
| 3-Methyl-2-hepten | 36,2 | 36,2 | 36,2 | 39,3 |
| Nachlauf | <0,01 | <0,01 | <0,01 | 0,1 |

| **Versuchsbedingungen** | | | | |
|---|---|---|---|---|
| Rh-Konzentration [ppm], bezogen auf Octeneinsatz | 10 | 10 | 10 | 10 |
| Äquivalente TPP | 3 | 50 | 100 | 3 |
| Druck [MPa] | 18 | 27 | 18 | 14 |
| Temperatur [°C] | 140 | 140 | 140 | 160 |
| Reaktionszeit [h] | 1 | 2 | 1 | 2 |

| **GC-Analyse Produkt (%)** | | | | |
|---|---|---|---|---|
| Vorlauf | 0,1 | 0,1 | 0,1 | 0,1 |
| C8 Kohlenwasserstoffe | 52,2 | 70,9 | 81,7 | 14,1 |
| Zwischenlauf | 0,8 | 0,1 | 0,1 | 1,9 |
| Iso-Nonanale | 45,7 | 28,3 | 17,6 | 76,1 |
| n-Nonanal | 0,5 | 0,1 | 0,1 | 0,5 |
| Nachlauf | 0,7 | 0,4 | 0,4 | 7,3 |

### III. Oxidation des nach Schritt II. erhaltenen Isononanals zu Isononansäure

Aus dem nach Beispiel IIa erhaltenen Isononanal wurden zunächst Leichtsieder und unumgesetztes Olefin als Kopfprodukt an einer 24 Bödenkolonne bei 200 hPa, einer Sumpftemperatur von 120°C und einem Rücklaufverhältnis von 2:1 abgetrennt. Nach Leichtsiederabtrennung wurde die Sumpftemperatur auf 140-150°C angezogen und das Isononanal über Kopf abgezogen (Siedepunkt °C bei 100 hPa: 110-114°C), während Hochsieder im Destillationssumpf verblieben.

Das erhaltene Isononanal wies folgende gaschromatographisch ermittelte Zusammensetzung sowie folgende Kennzahlen auf und wurde für die nachfolgende Flüssigphasenoxidation eingesetzt.

**Tabelle 3: Gaschromatographische Analyse (FI.-%, gemäß DIN 51405) des Isononanals ausgehend von 2-Ethylhexanol**

| | |
|---|---|
| Vorlauf/C8-Kohlenwasserstoffe | 0,2 |
| Zwischenlauf | 0,4 |
| 2-Ethyl-4-methylhexanal | 10,8 |
| 2-Propyl-3-methylpentanal | 3,6 |
| 2,5-Dimethylheptanal | 21,9 |
| 2,3-Dimethylheptanal (Isomer) | 4,8 |
| 2,3-Dimethylheptanal (Isomer) + 2-Ethylheptanal | 8,4 |
| 2-Methyloctanal | 1,7 |
| 3-Ethylheptanal | 10,4 |
| 4-Methyloctanal | 20,6 |
| 4,5-Dimethylheptanal | 0,6 |
| 6-Methyloctanal | 11,0 |
| andere i-Nonanale | 1,8 |
| n-Nonanal | 0,9 |
| Nachlauf | 2,9 |

**Tabelle 4: Kennzahlen des Isononanals ausgehend von 2-Ethylhexanol**

| Kennzahl / Einheit | DIN/ASTM | Wert |
|---|---|---|
| V₂₀ (mm²/s) | D 445 | 1,536 |
| V₄₀ (mm²/s) | | 1,179 |
| Erstarrungspunkt (°C) | -100 | -100 |
| d^{20/4} (g/cm³) d^{50/4} (g/cm³) | DIN 51757, Verf. D/ASTM D 4052 | 0,827 0,811 |
| n^{20/D} | DIN 51423-2/ ASTM D 1747 | 1,424 |
| CO-Zahl (mg KOH/g) | DIN 53173 | 339/349 |
| Flammpunkt (°C) | ISO 2719 | 60 |
| Platin/Kobalt-Farbzahl Hazen | DIN ISO 6271/ ASTM D 1209 | 15 |

Die Flüssigphasenoxidation des Isononanals zu Isononansäure erfolgte ohne Lösungsmittelzusatz in einem Blasensäulenreaktor bei 50°C mit reinem Sauerstoff bei Normaldruck über einen Zeitraum von 6 Stunden. Dem Einsatzaldehyd wurde eine 50 Gew.-%ige wässrige Lösung von Kaliumhydroxid in einer solchen Menge zugesetzt, dass pro Mol Isononanal 50 mmol Kalium vorlagen.

Die erhaltene Rohsäure wurde anschließend an einer 4,5 Bödenkolonne bei einer Sumpftemperatur von 148 bis 159°C und bei einer Kopftemperatur von 136-139°C bei 20 hPa destilliert. Leichtsieder und unumgesetzter Aldehyd wurden als Vorlauffraktion abgetrennt und Schwersieder verblieben im Destillationsrückstand. Die Destillationsausbeute an Isononansäure betrug 84,7% mit einer gaschromatographisch ermittelten Reinheit von 98,8%.

Die erhaltene Isononansäure wies folgende nach DIN 51405 gaschromatographisch ermittelte Zusammensetzung (FI.-%) auf:

**Tabelle 5: Gaschromatographische Analyse der Isononansäure ausgehend von 2-Ethylhexanol (FI.-%, gemäß DIN 51405)**

| | |
|---|---|
| Vorlauf | 0,4 |
| 2-Ethyl-4-methylhexansäure | 9,3 |
| 2-Propyl-3-methylpentansäure | 3,0 |
| 2,5-Dimethylheptansäure + 2,3-Dimethylheptansäure (Isomer) | 25,7 |
| 2,3-Dimethylheptansäure (Isomer) | 8,4 |
| 3-Ethylheptansäure + 2-Ethylheptansäure | 12,9 |
| 2-Methyloctansäure | 0,8 |
| 4-Methyloctansäure | 20,9 |
| 6-Methyloctansäure | 12,3 |
| n-Nonansäure | 0,3 |
| andere i-Nonansäuren | 5,2 |
| Nachlauf | 0,8 |

Die für die Isononansäure ermittelten Kennzahlen sind in der Tabelle 6 zusammengestellt.

**Tabelle 6: Kennzahlen der Isononansäure ausgehend von 2-Ethylhexanol**

| Kennzahl / Einheit | DIN/ASTM | Wert |
|---|---|---|
| V₂₀ (mm²/s) | D 445 | 10,68 |
| V₄₀ (mm²/s) | | 5,88 |
| d^{20/4} (g/cm³) | DIN 51757, Verf. D/ | 0,906 |
| d^{50/4} (g/cm³) | ASTM D 4052 | 0,883 |
| n^{20/D} | DIN 51 423-2/ ASTM D 1747 | 1,432 |
| Erstarrungspunkt (°C) | | -81 |
| Siedepunkt (°C) bei 1013 hPa | DIN 53171 / ASTM D 1078 | 241-242 |
| Säurezahl mg KOH/g | DIN EN ISO 2114/ ASTM D 1613 | 351 |
| Flammpunkt (°C) | ISO 2719 | 129 |
| Platin/Kobalt-Farbzahl Hazen | DIN ISO 6271/ ASTM D 1209 | 7 |

### IV. Vinylierung der nach Schritt III. erhaltenen Isononansäure

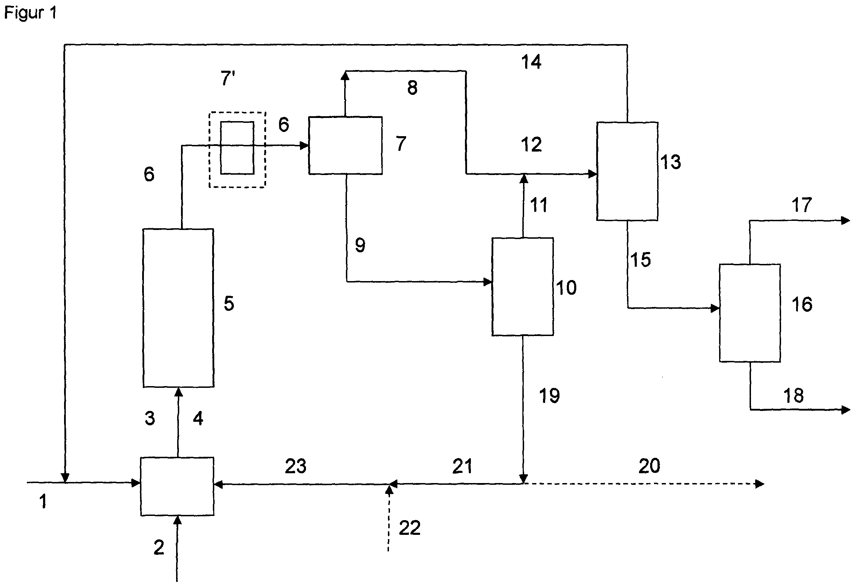

Für die Durchführung des nachfolgenden Beispiels IV./1 (kontinuierliche Verfahrensführung) wurde der Versuchsaufbau gemäß Figur 1 verwendet.

Das Transvinylierungsreagenz Vinylacetat wurde über die Leitung (1) und die zu vinylierende Isononansäure über die Leitung (2) in ein Mischgefäß (3) geführt, aus dem über Leitung (4) die Mischung in das als Strömungsrohr ausgestaltete Reaktionsgefäß (5) eingeleitet wurde. Der flüssige Reaktionsaustrag wurde über Leitung (6) auf ein Entspannungsgefäß (7) gegeben, in dem auf Normaldruck entspannt wurde, gegebenenfalls nach vorheriger Kühlung in der Kühleinrichtung (7') (gestrichelt dargestellt). Die bei dem Entspannungsvorgang gegebenenfalls gebildete Gasphase wurde über Leitung (8) abgeführt und die gebildete Flüssigphase wurde über Leitung (9) auf das Trenngefäß (10) gegeben. In dem Trenngefäß (10) erfolgte eine Aufspaltung in eine mit Vinylacetat, Essigsäure und dem gewünschten Vinylisononanoat angereicherte flüchtige Fraktion, die über Leitung (11) mit den gegebenenfalls über Leitung (8) herangeführten flüchtigen Anteilen aus der Entspannungsstufe vereinigt wurden und über Leitung (12) auf das Trenngefäß (13) geführt wurden. Das im Trenngefäß (13) abgetrennte Vinylacetat wurde über Leitung (14) zurückgeführt und mit dem über Leitung (1) herangeführten Vinylacetat vereinigt. Die im Trenngefäß (13) anfallende und während der Transvinylierungsreaktion gebildete Essigsäure sowie das gewünschte Vinylisononanoat wurden über Leitung (15) abgeführt und auf das Trenngefäß (16) gegeben, aus dem die gebildete Essigsäure über Leitung (17) und das gewünschte Vinylisononanoat über Leitung (18) abgezogen wurden. Das erhaltene Vinylisononoat konnte anschließend weiter aufgereinigt werden (nicht in Figur 1 gezeigt).

Der über Leitung (9) abgeführte flüssige Austrag wurde gaschromatographisch analysiert. Die aus den Analysendaten ermittelten Umsatz-, Selektivitäts- und Ausbeuteangaben sowie die daraus errechnete Raum-Zeit-Ausbeute an Vinylisononanoat sind in der nachfolgenden Tabelle 7 zusammengefasst. Ebenfalls sind die im Reaktionsgefäß (5) eingestellten Reaktionsbedingungen angegeben.

Die in dem Trenngefäß (10) erhaltene, schwerer flüchtige Fraktion, enthaltend die nicht umgesetzte Isononansäure mit dem Transvinylierungskatalysator wurde über Leitung (19) abgeführt und gegebenenfalis nach Ausschleusen eines hochsiederhaltigen Seitenstroms über Leitung (20) (gestrichelt dargestellt) als Katalysatorkreislauf über Leitung (21) zurückgeführt.

Die Katalysatorlösung wurde durch Abmischen der Katalysatorvorstufe Palladiumacetat Pd(OAc)₂ mit dem zweizähnigen, stickstoffhaltigen Liganden 1,10-Phenanthrolin in einer Mischung aus Vinylacetat und Isononansäure zubereitet und über Leitung (22) ergänzt. Die resultierende Mischung aus Alt- und Frischkatalysator wurde anschließend über Leitung (23) in das Mischgefäß (3) eingespeist.

**Tabelle 7: Bedingungen und Ergebnisse der kontinuierlichen Herstellung von Vinylisononanoat im Strömungsrohr**

| | |
|---|---|
| Beispiel Nr. | IV./1 |
| Verweilzeit [min] | 75 |
| Reaktorvolumen [ml] | 200 |
| Temperatur [°C] | 140 |
| Druck [MPa] | 2 |
| Molares Verhältnis Carbonsäure:Vinylacetat: Katalysatorvorstufe | 1,0:5,0:0,0010 |
| Molares Verhältnis Katalysatorvorstufe: Ligand | 1:8 |
| Katalysatorvorstufe | Pd(OAc)₂ |
| Ligand | 1,10-Phenanthrolin |
| Carbonsäure [g/h] | 39,6 |
| Vinylacetat [g/h] | 107,8 |
| Katalysatorvorstufe [mg/h] | 56,2 |
| Ligand [mg/h] | 361,1 |
| Umsatz [%] | 77,2 |
| Ausbeute [%] | 76,7 |
| Selektivität [%] | 99,3 |
| Raum-Zeit-Ausbeute [g/L.h] | 177 |

Das erhaltene Vinylestergemisch wurde anschließend an einer 4,5 Bödenkolonne bei einer Sumpftemperatur von 86 bis 160°C und bei einer Kopftemperatur von 68-95°C bei 5-500 hPa destilliert. Essigsäure und nicht umgesetztes Vinylacetat wurden als Vorlauffraktion abgetrennt und die nicht umgesetzte Isononansäure, der Palladium-Komplex und freier Ligand verblieben im Destillationsrückstand.

Das erhaltene Vinylisononanoat wies folgende nach DIN 51405 gaschromatographisch ermittelte Zusammensetzung (FI.-%) auf:

**Tabelle 7a: Gaschromatographische Analyse des Vinylisononanoats ausgehend von 2-Ethylhexanol (FI.-%, gemäß DIN 51405)**

| | |
|---|---|
| Vorlauf | 0,0 |
| Zwischenlauf | 0,1 |
| Vinyl-2-ethyl-4-methylhexanoat | 7,3 |
| Vinyl-2-propyl-3-methylpentanoat | 2,3 |
| Vinyl-2,3-dimethylheptanoat + Vinyl-2,5-dimethylheptanoat | 44,5 |
| Vinyl-3-ethylheptanoat | 16,6 |
| Vinyl-4-methyloctanoat | 17,4 |
| Vinyl-6-methyloctanoat | 3,9 |
| stellungsisomere aliphatische Vinylester | 7,9 |
| Nachlauf | 0 |

Die für das Vinylisononanoat ermittelten Kennzahlen sind in der Tabelle 7b zusammengestellt.

**Tabelle 7b: Kennzahlen des Vinylisononanoats ausgehend von 2-Ethylhexanol**

| Kennzahl /Einheit | DIN/ASTM | Wert |
|---|---|---|
| V₂₀ (mm²/s) | D445 | 1,735 |
| V₄₀ (mm²/s) | | 1,271 |
| d^{20/4} (g/cm³) | DIN 51757, Verf. D/ ASTM D 4052 | 0,8776 |
| | | 0,8516 |
| n^{20/D} | DIN 51 423-2/ ASTM D 1747 | 1,4323 |
| Erstarrungspunkt (°C) | | -100 |
| Säurezahl (mg KOH/g) | DIN EN ISO 2114/ ASTM D 1613 | 0,57 |
| Flammpunkt (°C) | ISO 2719 | 77 |
| Platin/Kobalt-Farbzahl Hazen | DIN ISO 6271/ ASTM D 1209 | 6 |

Die nachfolgenden Beispiele IV./2-4 wurden absatzweise in einem geschlossenen Reaktionsgefäß durchgeführt. Die gemäß Schritt III. hergestellte Isononansäure, Vinylacetat, Katalysatorstufe und Ligand wurden in einen 1 L-Autoklaven gegeben. Der Autoklav wurde mit 600 Umdrehungen pro Minute gerührt und auf die jeweils angegebene Reaktionstemperatur gebracht. Durch Aufpressen von Stickstoff wurde ein Druck von 2 MPa eingestellt.

Nach der angegebenen Reaktionszeit ließ man den Ansatz abkühlen und entspannte auf Normaldruck. Das erhaltene Reaktionsgemisch wurde gaschromatographisch analysiert und der Umsatz an Isononansäure sowie die Selektivität und Ausbeute an Vinylisononanoat bestimmt. Die Reaktionsbedingungen und Resultate der diskontinuierlichen Herstellung von Vinylisononanoat sind in der nachfolgenden Tabelle 8 zusammengestellt.

**Tabelle 8: Bedingungen und Ergebnisse der diskontinuierlichen Herstellung von C₉-Vinylestern im Autoklaven**

| **Beispiel Nr.** | **IV./2** | **IV./3** | **IV./4** |
|---|---|---|---|
| Reaktionszeit [min] | 90 | 120 | 180 |
| Temperatur [°C] | 130 | 140 | 100 |
| Molares Verhältnis C9-Säure:Vinylacetat: | 1,0:3,0: | 1,0:6,0: | 1,0:5,0: |
| Katalysatorvorstufe | 0,00125 | 0,00200 | 0,00125 |
| Molares Verhältnis | | | |
| Katalysatorvorstufe: Ligand | 1:5 | 1:8 | 1:5 |
| Katalysatorvorstufe | Pd(OAc)₂ | Pd(OAc)₂ | Pd(OAc)₂ |
| Ligand | 1,10-Phenanthrolin | 2,2'-Bipyridyl | 1,10-Phenanthrolin |
| C9-Säure [g] | 122,1 | 75,7 | 86,7 |
| Vinylacetat [g] | 199,3 | 247,2 | 235,9 |
| Katalysatorvorstufe [mg] | 211,6 | 214,9 | 153,8 |
| Ligand [mg] | 869,2 | 1196,2 | 617,2 |
| Umsatz [%] | 79,5 | 83,1 | 49,8 |
| Ausbeute [%] | 78,9 | 82,2 | 49,4 |
| Selektivität [%] | 99,2 | 98,9 | 99,1 |

### V. Vinylierung eines Säuregemisches enthaltend tertiäre Isononansäuren

Für die nachfolgende Transvinylierung wurde ein Säuregemisch enthaltend tertiäre Isononansäuren eingesetzt, das aus der Kochreaktion gemäß J. Falbe, Carbon Monoxide in Organic Synthesis, Springer Verlag Berlin, Heidelberg, New York, 1970, Seiten 127-135 mit dem zuvor genannten Octeneinsatzgemisch aus Schritt I. erhalten wurde und folgende gaschromatographisch ermittelte Zusammensetzung aufwies (FI.-%, gemäß DIN 51405):

| | |
|---|---|
| Vorlauf + C8-Olefine | 0,1 |
| Octen-Oligomere | 0,1 |
| Zwischenlauf | 0,1 |
| 2,2-Dimethylheptansäure | 13,1 |
| 2-Ethyl-2-methylhexansäure | 81,5 |
| stellungsisomere aliphatische Isononansäuren | 4,8 |
| Nachlauf | 0,3 |

Der Versuchsaufbau wurde gemäß Beispiel IV./1 verwendet. Die aus den Analysendaten ermittelten Umsatz-, Selektivitäts- und Ausbeuteangaben sowie die daraus errechnete Raum-Zeit-Ausbeute an Vinylisononanoat sind in der nachfolgenden Tabelle 9 zusammengefasst. Ebenfalls sind die im Reaktionsgefäß eingestellten Reaktionsbedingungen angegeben.

**Tabelle 9: Bedingungen und Ergebnisse der kontinuierlichen Transvinylierung von einem Gemisch enthaltend tertiäre Isononansäuren im Strömungsrohr**

| Beispiel Nr. | IV./5 |
|---|---|
| Verweilzeit [min] | 60 |
| Reaktorvolumen [ml] | 200 |
| Temperatur [°C] | 140 |
| Druck [MPa] | 2 |
| Molares Verhältnis Carbonsäure:Vinylacetat: Katalysatorvorstufe | 2,0:1,0:0,00244 |
| Molares Verhältnis Katalysatorvorstufe:Ligand | 1:3 |
| Katalysatorvorstufe | Pd(OAc)₂ |
| Ligand | 1,10-Phenanthrolin |
| Carbonsäure [g/h] | 142,5 |
| Vinylacetat [g/h] | 38,8 |
| Katalysatorvorstufe [mg/h] | 246,7 |
| Ligand [mg/h] | 594,0 |
| Umsatz [%]^{[a]} | 80,7 |
| Ausbeute [%]^{[a]} | 70,0 |
| Selektivität [%] | 86,8 |
| Raum-Zeit-Ausbeute [g/L·h] | 291 |

| | |
|---|---|
| ^{[a]} Umsatz und Ausbeute bezüglich Vinylacetat | |

Das erhaltene Vinylestergemisch wurde anschließend an einer 9,0 Bödenkolonne bei einer Sumpftemperatur von 75 bis 140°C und bei einer Kopftemperatur von 34-114°C bei 10-1013 hPa destilliert. Essigsäure und nicht umgesetztes Vinylacetat wurden als Vorlauffraktion abgetrennt und die nicht umgesetzten tertiären Isononansäuren, der Palladium-Komplex und freier Ligand verblieben im Destillationsrückstand.

Das erhaltene Vinylester-Gemisch hat laut gaschromatographischer Analyse (FI.-%, gemäß DIN 51405) eine Reinheit von 99,7%, der Rest auf 100% besteht aus Vorlauf- und Nachlaufkomponenten. Das Vinylester-Gemisch (99,7%) besteht hauptsächlich aus den Isomeren Vinyl-2-ethyl-2-methyl-hexanoat (90,4%) und Vinyl-2,2-dimethylheptanoat (4,8%) sowie mit Rest auf 99,7% aus weiteren Vinylestern stellungsisomerer Isononansäuren. Die für das Vinylisononanoat ermittelten Kennzahlen sind in der Tabelle 10 zusammengestellt.

**Tabelle 10: Kennzahlen des Vinylisononanoats aus der kontinuierlichen Transvinylierung von einem Gemisch enthaltend tertiäre Isononansäuren**

| Kennzahl / Einheit | DIN/ASTM | Wert |
|---|---|---|
| V₂₀ (mm²/s) | D 445 | 1,752 |
| d^{20/4} (g/cm³) | DIN 51757, Verf. D/ ASTM D 4052 | 0,8776 |
| n^{20/D} | DIN 51 423-2/ ASTM D 1747 | 1,4313 |
| Säurezahl (mg KOH/g) | DIN EN ISO 2114/ ASTM D 1613 | 0,046 |
| Platin/Kobalt-Farbzahl Hazen | DIN ISO 6271/ ASTM D 1209 | 13 |

## Patentansprüche

1. Verfahren zur Herstellung von einem Vinylester eines Gemisches von Isononansäuren ausgehend von 2-Ethylhexanol, **dadurch gekennzeichnet, dass** man
(a) 2-Ethylhexanol in Gegenwart eines Katalysators zu einem Octengemisch dehydratisiert;
(b) das nach Schritt a) erhaltene Octengemisch in Gegenwart einer Übergangsmetallverbindung der Gruppe VIII des Periodensystems der Elemente mit Kohlenmonoxid und Wasserstoff zu einem Gemisch isomerer Isononanale umsetzt und anschließend zu einem Gemisch von Isononansäuren oxidiert, oder mit Kohlenmonoxid in Gegenwart von Wasser zu einem Gemisch von Isononansäuren umsetzt, und
(c) das nach Schritt b) erhaltene Gemisch von Isononansäuren zu dem entsprechenden Vinylester umsetzt durch Umsetzung mit Acetylen oder durch Umsetzung mit einem Vinylester einer anderen Carbonsäure.

2. Verfahren gemäß Anspruch 1, **dadurch gekennzeichnet, dass** man in Schritt a) als Katalysator Aluminiumoxid, Nickel niedergeschlagen auf Aluminiumoxid, oder Phosphorsäure niedergeschlagen auf Siliziumdioxid oder Aluminiumoxid verwendet.

3. Verfahren gemäß Anspruch 1 oder 2, **dadurch gekennzeichnet, dass** man in Schritt a) 2-Ethylhexanol in der Gasphase dehydratisiert.

4. Verfahren gemäß einem oder mehreren der Ansprüche 1 bis 3, **dadurch gekennzeichnet, dass** man in Schritt b) als Übergangsmetallverbindung der Gruppe VIII des Periodensystems der Elemente eine Kobalt- oder Rhodiumverbindung verwendet.

5. Verfahren gemäß einem oder mehreren der Ansprüche 1 bis 4, **dadurch gekennzeichnet, dass** man in Schritt b) das Gemisch isomerer Isononanale destilliert.

6. Verfahren gemäß einem oder mehreren der Ansprüche 1 bis 5, **dadurch gekennzeichnet, dass** man in Schritt b) das Gemisch isomerer Isononanale in Anwesenheit von Alkali- oder Erdalkalimetallcarboxylaten zu dem Gemisch von Isononansäuren oxidiert.

7. Verfahren gemäß Anspruch 6, **dadurch gekennzeichnet, dass** man als Alkali- oder Erdalkalimetallcarboxylat Lithium-, Kalium-, Natrium-, Calcium- oder Bariumisononanoat verwendet.

8. Verfahren gemäß einem oder mehreren der Ansprüche 1 bis 7, **dadurch gekennzeichnet, dass** man in Schritt b) das Gemisch isomerer Isononanale mit Sauerstoff oder sauerstoffenthaltenden Gasen zu dem Gemisch von Isononansäuren oxidiert.

9. Verfahren gemäß Anspruch 1, **dadurch gekennzeichnet, dass** die Umsetzung des Gemisches von Isononansäuren mit Acetylen in Gegenwart von Zinksalzen erfolgt.

10. Verfahren gemäß Anspruch 1, **dadurch gekennzeichnet, dass** in Schritt c) das Gemisch von Isononansäuren mit Vinylacetat oder Vinylpropionat umgesetzt wird.

11. Verfahren gemäß Anspruch 10, **dadurch gekennzeichnet, dass** die bei der Umsetzung des Gemisches von Isononansäuren mit Vinylacetat freigesetzte Essigsäure zur Herstellung von Vinylacetat, Acetatestern, Acetylhalogeniden, Amiden, Carbonsäureanhydriden, Chloressigsäuren oder Ethanol verwendet wird.

## Claims

1. Process for preparing a vinyl ester of a mixture of isononanoic acids starting out from 2-ethylhexanol, **characterized in that**
(a) 2-ethylhexanol is dehydrated in the presence of a catalyst to form an octene mixture;
(b) the octene mixture obtained in step a) is reacted with carbon monoxide and hydrogen in the presence of a transition metal compound of group VIII of the Periodic Table of the Elements to form a mixture of isomeric isononanals and subsequently oxidized to a mixture of isononanoic acids, or reacted with carbon monoxide in the presence of water to form a mixture of isononanoic acids; and
(c) the mixture of isononanoic acids obtained in step b) is converted into the corresponding vinyl ester by reaction with acetylene or by reaction with a vinyl ester of another carboxylic acid.

2. Process according to Claim 1, **characterized in that** aluminium oxide, nickel deposited on aluminium oxide, or phosphoric acid deposited on silicon dioxide or aluminium oxide is used as catalyst in step a).

3. Process according to Claim 1 or 2, **characterized in that** 2-ethylhexanol is dehydrated in the gas phase in step a).

4. Process according to one or more of Claims 1 to 3, **characterized in that** a cobalt compound or rhodium compound is used as transition metal compound of group VIII of the Periodic Table of the Elements in step b).

5. Process according to one or more of Claims 1 to 4, **characterized in that** the mixture of isomeric isononanals is distilled in step b).

6. Process according to one or more of Claims 1 to 5, **characterized in that** the mixture of isomeric isononanals is oxidized to the mixture of isononanoic acids in the presence of alkali metal or alkaline earth metal carboxylates in step b).

7. Process according to Claim 6, **characterized in that** lithium isononanoate, potassium isononanoate, sodium isononanoate, calcium isononanoate or barium isononanoate is used as alkali metal or alkaline earth metal carboxylate.

8. Process according to one or more of Claims 1 to 7, **characterized in that** the mixture of isomeric isononanals is oxidized to the mixture of isononanoic acids by means of oxygen or oxygen-containing gases in step b).

9. Process according to Claim 1, **characterized in that** the reaction of the mixture of isononanoic acids with acetylene is carried out in the presence of zinc salts.

10. Process according to Claim 1, **characterized in that** the mixture of isononanoic acids is reacted with vinyl acetate or vinyl propionate in step c).

11. Process according to Claim 10, **characterized in that** the acetic acid liberated in the reaction of the mixture of isononanoic acids with vinyl acetate is used for preparing vinyl acetate, acetate esters, acetyl halides, amides, carboxylic anhydrides, chloroacetic acids or ethanol.

## Revendications

1. Procédé de préparation d'un ester vinylique d'un mélange d'acides isononanoïques à partir de 2-éthylhexanol, **caractérisé en ce que**
(a) on déshydrate du 2-éthylhexanol en présence d'un catalyseur pour obtenir un mélange d'octènes ;
(b) on fait réagir le mélange d'octènes obtenu dans l'étape a), en présence d'un composé d'un métal de transition du Groupe VIII du Système Périodique des Eléments, avec du monoxyde de carbone et de l'hydrogène pour obtenir un mélange d'isononanals isomères, puis on l'oxyde pour obtenir un mélange d'acides isononanoïques, ou on le fait réagir avec du monoxyde de carbone en présence d'eau pour obtenir un mélange d'acides isononanoïques, et
(c) on fait réagir le mélange d'acides isononanoïques obtenu dans l'étape b) pour obtenir l'ester vinylique correspondant, par réaction avec de l'acétylène ou par réaction avec un ester vinylique d'un autre acide carboxylique.

2. Procédé selon la revendication 1, **caractérisé en ce qu'**on utilise dans l'étape a) en tant que catalyseur de l'oxyde d'aluminium, du nickel précipité sur de l'oxyde d'aluminium, ou de l'acide phosphorique précipité sur du dioxyde de silicium ou de l'oxyde d'aluminium.

3. Procédé selon la revendication 1 ou 2, **caractérisé en ce qu'**on déshydrate le 2-éthylhexanol dans l'étape a) en phase gazeuse.

4. Procédé selon l'une ou plusieurs des revendications 1 à 3, **caractérisé en ce qu'**on utilise dans l'étape b) en tant que composé d'un métal de transition du Groupe VIII du Système Périodique des Eléments un composé du cobalt ou du rhodium.

5. Procédé selon l'une ou plusieurs des revendications 1 à 4, **caractérisé en ce qu'**on distille dans l'étape b) le mélange d'isononanals isomères.

6. Procédé selon l'une ou plusieurs des revendications 1 à 5, **caractérisé en ce qu'**on oxyde dans l'étape b) le mélange d'isononanals isomères en présence de carboxylates d'un métal alcalin ou d'un métal alcalino-terreux, pour obtenir le mélange d'acides isononanoïques.

7. Procédé selon la revendication 6, **caractérisé en ce qu'**on utilise en tant que carboxylate d'un métal alcalin ou d'un métal alcalino-terreux de l'isononanoate de lithium, de potassium, de sodium, de calcium ou de baryum.

8. Procédé selon l'une ou plusieurs des revendications 1 à 7, **caractérisé en ce qu'**on oxyde dans l'étape b) le mélange d'isononanals isomères avec de l'oxygène ou des gaz oxygénés, pour obtenir les mélanges d'acides isononanoïques.

9. Procédé selon la revendication 1, **caractérisé en ce que** la réaction du mélange d'acides isononanoïques avec l'acétylène a lieu en présence de sels de zinc.

10. Procédé selon la revendication 1, **caractérisé en ce que**, dans l'étape c), on fait réagir le mélange d'acides isononanoïques avec de l'acétate de vinyle ou du propionate de vinyle.

11. Procédé selon la revendication 10, **caractérisé en ce qu'**on utilise l'acide acétique libéré lors de la réaction du mélange d'acides isononanoïques avec de l'acétate de vinyle pour préparer de l'acétate de vinyle, des esters acétates, des halogénures d'acétyle, des amides, des anhydrides carboxyliques, des acides chloracétiques ou de l'éthanol.
